Europäisches Patentamt

European Patent Office

Office européen des brevets

(11) Veröffentlichungsnummer: **0 343 544**

**A2**

(12) **EUROPÄISCHE PATENTANMELDUNG**

(21) Anmeldenummer: 89109115.9

(22) Anmeldetag: 20.05.89

(51) Int. Cl.⁴: **A61K 35/74** , **A61K 45/05** , **A61K 37/20**

(30) Priorität: 26.05.88 DE 3817762

(43) Veröffentlichungstag der Anmeldung:
29.11.89 Patentblatt 89/48

(84) Benannte Vertragsstaaten:
AT BE CH DE ES FR GB GR IT LI LU NL SE

(71) Anmelder: SANUM-KEHLBECK GmbH & Co. KG
Bahnhofstrasse 2
D-2812 Hoya(DE)

(72) Erfinder: Pulverer, Gerhard
Mohnweg 25
D-5000 Köln 40(DE)

(74) Vertreter: Werner, Hans-Karsten, Dr. et al
Deichmannhaus am Hauptbahnhof
D-5000 Köln 1(DE)

(54) **Immunstimulierendes Mittel aus Propionibakterien sowie Verfahren zur Herstellung desselben.**

(57) Es wird ein immunstimulierendes Mittel aus Propionibakterien, insbesondere aus den Stämmen Propionibacterium granulosum DSM 1773, Propionibacterium avidum DSM 1772 und/oder Propionibacterium acnes ATCC 6919, beschrieben, welches dadurch gekennzeichnet ist, daß es aus dem wäßrigen Zell-Lysat, vorzugsweise aus dem mit organischen Lösungsmitteln extrahierten Zell-Lysat besteht und gewünschtenfalls dialysiert und gefriergetrocknet ist, sowie ein Verfahren zu dessen Herstellung.

EP 0 343 544 A2

**Immunstimulierendes Mittel aus Propionibakterien sowie Verfahren zur Herstellung desselben**

Gegenstand der vorliegenden Erfindung ist ein immunstimulierendes Mittel aus Propionibakterien, insbesondere aus den Stämmen Propionibacterium granulosum DSM 1773, Propionibacterium avidum DSM 1772 und/oder Propionibacterium acnes ATCC 6919, und ein Verfahren zur Herstellung desselben.

Als immunstimulierende Mittel sind Zytokine wie Interferone, Interleukine und andere, von Lymphozyten sezernierte Stoffe, sogenannte "biological response modifiers" (BRM) bekannt. Gegenstand der DE-PS 30 11 461 sind Zellwand- und Ganzzellpräparate auf der Grundlage von abgetöteten ganzen Zellen bestimmter Propionibakterien, die für die Radio- und Chemotherapie von Tumoren geeignet sind. Ein Nachteil dieser Zellpräparate ist die Tatsache, daß die Verabreichung der Zellbestandteile zu Nebenreaktionen führen kann. Des weiteren ist eine beliebige Dosierung aufgrund des heterogenen Aufbaus dieser Präparate nur schwer möglich.

Überraschenderweise wurde gefunden, daß wäßrige Lysate aus Propionibakterien, insbesondere aus den Stämmen Propionibacterium granulosum DSM 1773, Propionibacterium avidum DSM 1772 und/oder Propionibacterium acnes ATCC 6919, eine immunstimulierende Wirkung zeigen. Nach der Extraktion des Rohlysats mit organischen Lösungsmitteln, insbesondere nach Phenolextraktion, bleibt die wirksame Komponente in der wäßrigen Phase. Besonders bevorzugt ist die Extraktion mit wäßrigen Phenollösungen. Durch geeignete Reinigungsverfahren, insbesondere chromatographische Trennung an Gelpermeationsmaterialien wie Sepharose$^R$ CL-6B, lassen sich aus dem wäßrigen Lysat bestimmte Fraktionen anreichern, die Lipoteichonsäuren enthalten. Diese Fraktionen sind wirksame Immunstimulatoren. Das wäßrige Lysat oder die wirksamen Fraktionen können gefriergetrocknet werden. Dabei tritt ein Wirksamkeitsverlust nicht ein. Gewünschtenfalls können das wäßrige Lysat oder die wirksamen Fraktionen vorher dialysiert worden sein. Das erfindungsgemäße Mittel kann neben den wirksamen Bestandteilen auch die üblichen pharmazeutischen Träger und Hilffstoffe enthalten.

Das erfindungsgemäße Mittel ist erhältlich durch Aufschluß der betreffenden Bakterienzellen, vorzugsweise durch mechanisches Zerstören der Zellen, Abtrennen der Zelltrümmer und Sammeln des wäßrigen Lysats. Es ist vorteilhaft, das gewonnene Lysat weiter aufzuarbeiten, beispielsweise durch weitere Zentrifugationsschritte. Zwischen den Zentrifugationsschritten ist es vorteilhaft, das wäßrige Lysat im Dampftopf zu erhitzen. Gewünschtenfalls schließt sich nach einer letzten Ultrafiltration eine Gefriertrocknung an. In einer vorteilhaften Ausgestaltung des Verfahrens werden die Zellen mit phenolischer Lösung, vorzugsweise 95% Phenol gemischt und einige Stunden, vorzugsweise 1 bis 3 Stunden bei Raumtemperatur, gewünschtenfalls unter Rühren, behandelt. Nach einem Zentrifugationsschritt wird die wäßrige Phase gesammelt und danach gefriergetrocknet. Die wäßrige Phase des Lysats kann vor der Gefriertrocknung jedoch auch gegen Aqua destillata dialysiert werden.

Die auf dem oben beschriebenen Wege erhältlichen Rohextrakte können durch geeignete Maßnahmen in ihre wirksamen Bestandteile aufgetrennt werden, zum Beispiel durch Gelchromatographie an einer Matrix, welche bevorzugt eine Aufschlußgrenze des Molekulargewichts von ca. $4 \times 10^5$ aufweist. Geeignet sind zum Beispiel quervernetzte Agarosegele. Als immunstimulierend erweisen sich die Fraktionen, welche Lipoteichonsäuren (LTA) enthalten.

Die Lipoteichonsäuren (LTA) enthaltenden Fraktionen werden gesammelt, gegebenenfalls dialysiert und gefriergetrocknet.

Das erfindungsgemäße Mittel ist zur Verwendung als Immunstimulans geeignet. So kann das erfindungsgemäße Mittel beispielsweise zur Unterstützung der Krebstherapie und/oder -prophylaxe sowie der Rezidiv- und Metastasenvorbeugung verwendet werden. Es ist auch zur medikamentösen Unterstützung bei chirurgischen Eingriffen geeignet. Das erfindungsgemäße Mittel kann insbesondere zur Bekämpfung und Prophylaxe viraler, mykologischer und bakterieller Infektionen bzw. parasitologischer Erkrankungen verwendet werden.

Die Applikation des erfindungsgemäßen Mittels kann auf parenteralem Wege erfolgen. Es sind subkutane und nasale Verabreichung wie auch eine Applikation in Verbindung mittels Injektions- oder Infusionslösungen möglich. Orale Applikation ist neben der Applikation als Suppositorium ebenfalls möglich.

Die Abbildung 1 zeigt die Aktivität menschlicher Granulozyten, die mit Zymosan oder unterschiedlichen Konzentrationen von Propionibacterium-Lysat inkubiert wurden, gemessen mit der Chemilumineszenz-Methode.

O——O——O  Kontrollsubstanz (Zymosan)

●——●——●  KP-40-Lysat 2,5 mg/ml

△——△——△  KP-40-Lysat   5 mg/ml

Die Abbildung 2 zeigt die Aktivität menschlicher Monozyten, die mit Zymosan oder unterschiedlichen Konzentrationen von Propionibacterium-Lysat inkubiert wurden, gemessen mit der Chemilumineszenz-Methode.

O——O——O  Kontrollsubstanz (Zymosan)

●——●——●  KP-40-Lysat 2,5 mg/ml

△——△——△  KP-40-Lysat   5 mg/ml

Das KP-40-Lysat stammt aus Propionibacterium avidum. Ein Lysat (KP-45) aus Propionibacterium granulosum zeigte ähnliche Ergebnisse.

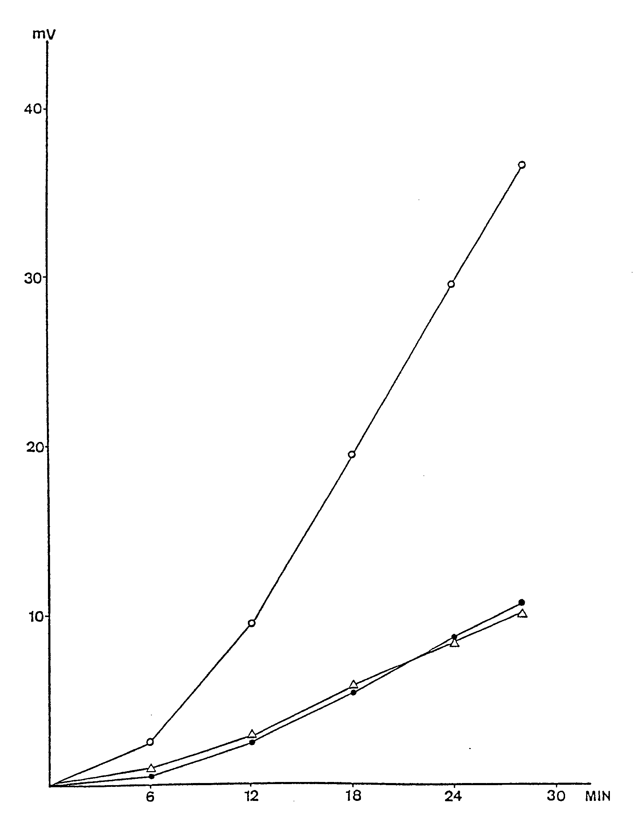

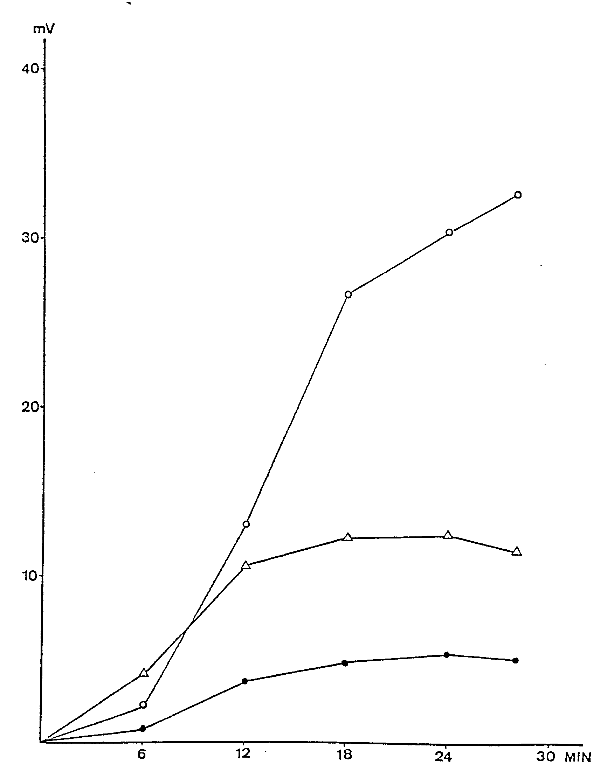

Die immunomodulatorische Wirkung des Lysats wurde durch die Untersuchung der Phagozytoseaktivität menschlicher Granulozyten, Monozyten und muriner Peritonealmakrophagen mit dem Chemilumineszenz-Test ermittelt. Dieser Test ist außerordentlich empfindlich und liefert verläßliche, gut reproduzierbare Ergebnisse. Wie in den Abbildungen 1 und 2 dargestellt, wurden menschliche Phagozyten (Granulozyten und Monozyten) in ihrer Aktivität beeinflußt, wenn sie mit unterschiedlichen Konzentrationen von Propionibacterium KP-40-Lysat inkubiert wurden. Konzentrationen von 2,5 und 5 mg/ml dieser Substanz bewirkten eine gesteigerte Aktivierbarkeit der Granulozyten und Monozyten, die aufgrund der vollständigen Löslichkeit des Lysats allerdings nicht so ausgeprägt war wie durch entsprechende Konzentrationen einer Zymosansuspension. Dieser Befund war aufgrund der unterschiedlichen Aktivierungsmedien (Lysatlösung bzw. Zymosansuspension) zu erwarten und stimmt mit früheren Untersuchungen überein, die eine stärkere Stimulierung von Phagozyten durch partikuläre Medien im Vergleich mit löslichen offenbarten.

Eine weitere Granulozytenfunktion wurde anhand des Chemotaxis-Testes ermittelt. Konzentrationen von 2,5 und 5 mg/ml des Lysats hatten keinen Einfluß auf die chemotaktische Beweglichkeit der Zellen.

Um diese Unterschiede hinsichtlich der Aktivatorsubstanz (Lösung bzw. Suspension) auszuschalten, wurde einer Versuchsgruppe von Balb/c-Mäusen (n = 5) Propionibacterium KP-40-Lysat (1, 2,5 und 5 mg) 7, 4 und 2 Tage vor Versuchsbeginn, in diesem Falle vor Entnahme von Peritonealmakrophagen, appliziert. Eine gleich große Kontrollgruppe erhielt physiologische Kochsalzlösung (gleiche Dosierungsmengen, -intervalle und Applikationsart). Chemilumineszenz-Studien mit den Peritonealmakrophagen und serumaktiviertem Zymosan offenbarten, daß die Lysat-Vorbehandlung eine massiv verstärkte Aktivierbarkeit der Zellen bewirkte. Dosisabhängig konnte eine Aktivierung von Peritonealmakrophagen nach intraperitonealer (+ 386% bei Applikation von 5 mg) und subkutaner (+ 127% bei Applikation von 5 mg) Gabe des Lysats nachgewiesen werden, was auf die gute immunstimulierende Wirksamkeit dieser Substanz erste Hinweise gab (Tabelle 1).

Tabelle 1

| | | Chemilumineszenz | |
|---|---|---|---|
| | | (mV) | (%) |
| Kontrolle | | 22 | 100 |
| 1 mg | i.p. | 73 | 332 |
| | s.c. | 29 | 131 |
| | nasal | 30 | 136 |
| 2,5 mg | i.p. | 81 | 368 |
| | s.c. | 31 | 140 |
| 5 mg | i.p. | 107 | 486 |
| | s.c. | 50 | 227 |
| Einfluß einer dreimaligen KP-40-Lysat-Applikation auf die Peritonealmakrophagenaktivität von Balb/c-Mäusen. Das Lysat wurde 7, 4 und 2 Tage vor Entnahme der Zellen intraperitoneal (i.p.), subkutan (s.c.) oder nasal verabreicht. | | | |

Von besonderem Interesse war die Beobachtung, daß auch die subkutane Applikation des löslichen Lysats eine verstärkte Aktivierbarkeit der Peritonealmakrophagen bewirkte. Dieser Befund ist auf die systemische Wirksamkeit dieser Substanz (trotz nicht-systemischer Applikation) zurückzuführen und könnte für die zukünftige Verwendung beim Menschen von besonderer Bedeutung sein.

Diese Daten führten zu der Überlegung, das immunstimulierende Lysat Balb/c-Mäusen lokal zu verabreichen, um dessen eventuelle systemische Wirksamkeit zu ermitteln. Chemilumineszenz-Experimente bestätigen die Vermutung, daß auch die lokale Applikation systemisch wirksam ist, und offenbaren eine verstärkte Aktivierbarkeit von Peritonealmakrophagen nach nasaler Lysatapplikation (7, 4 und 2 Tage vor deren Entnahme). Auch die Bestimmung des Milzgewichtes der Versuchstiere offenbarte, daß eine lokale (nasale) Lysat-Gabe dosisabhängig eine starke Zunahme (+ 71%) bewirkte, was als (sensibler) Parameter für die immunstimulatorische Potenz der Substanz gewertet werden kann. Ebenso bewirkte die dreimalige intraperitoneale und subkutane Gabe (dosisabhängig) eine starke Milzgewichtszunahme, die bei subkutaner Applikation am deutlichsten war (Tabellen 1 und 2).

| | | | mg Milz- gewicht/ 20 g Maus | (±SD) | (%) | (p) |
|---|---|---|---|---|---|---|
| Kontrolle | | | 66 | ±10,3 | 100 | |
| Experi- | | i.p. | 98 | ±15,2 | 148 | <0,05 |
| mentelle | 1 mg | s.c. | 107 | ± 6,2 | 162 | <0,01 |
| Gruppe | | nasal | 97 | ± 3,5 | 147 | <0,0125 |

| | | | mg Milz- gewicht/ 20 g Maus | (±SD) | (%) | (p) |
|---|---|---|---|---|---|---|
| **Kontrolle** | | | 66 | ±10,3 | 100 | |
| Experimentelle Gruppe | 2,5 mg | i.p. | 110 | ± 8,5 | 167 | <0,01 |
| | | s.c. | 134 | ± 2,2 | 200 | <0,005 |
| | | nasal | - | - | - | - |
| | 5 mg | i.p. | 130 | ± 5,9 | 197 | <0,005 |
| | | s.c. | 147 | ± 6,6 | 223 | <0,005 |
| | | nasal | 113 | ± 6,0 | 171 | <0,005 |

Tabelle 2:

Einfluß einer dreimaligen KP-40-Lysat-Applikation auf das Milzgewicht von Balb/c-Mäusen (5 pro Gruppe). Das Lysat wurde 7, 4 und 2 Tage vor der Milzextirpation intraperitoneal (i.p.), subkutan (s.c.) oder nasal verabreicht.

SD = Standardabweichung

p < 0,05 ist signifikant (student's t-test)

Die gesicherte Aussage über die immunstimulatorische Potenz einer Substanz gestattet deren tumorprotektive Wirkung in vivo. Aus diesem Grunde wurden Lysat-behandelten (und unbehandelten Mäusen = Kontrollgruppe) Balb/c-Mäusen L-1 Sarkomzellen intravenös inokuliert. Wie aus Tabelle 3 ersichtlich, führte die dreimalige Lysat-Applikation (5 mg intraperitoneal oder subkutan) zu einer signifikanten (p < 0,05) Reduktion von Lungenmetastasen. Diese hochgradige Reduktion der L-1 Sarkommetastasen in den Versuchstierlungen korreliert sehr gut mit der Zunahme des Milzgewichtes und mit den chemiluminometrisch ermittelten Aktivitätssteigerungen von Peritonealmakrophagen, Monozyten und Granulozyten und demonstriert eindrucksvoll die immunstimulatorische Wirksamkeit des Propionibacterium KP-40-Lysats.

Tabelle 3

| Je 10 Balb/c-Mäuse injiziert mit | $5 \times 10^4$ L-1 Sarkomzellen i.v. | | | |
|---|---|---|---|---|
| | $\bar{x}$ | % | ±SD | p |
| PBS (Kontrolle) | 381 | 100 | ± 73,9 | |
| Lysat (5 mg, s.c.) | 72 | 19 | ± 15,4 | <0,05 |
| Lysat (5 mg, i.p.) | 75 | 20 | ± 14,7 | <0,05 |

Durchschnittliche Anzahl von Lungenmetastasen (X) nach intravenöser (i.v.) Inokulation von $5 \times 10^4$ L-1 Sarkomzellen in Balb/c-Mäuse, die 7, 4 und 2 Tage vor Tumorzellinokulation mit 5 mg Lysat (subkutan (s.c.) oder intraperiton al (i.p.)) behandelt wurden.
SD = Standardabweichung
p < 0,05 ist signifikant

Gaschromatographische Analysen des Lysats offenbarten eine Vielzahl von Kohlenhydraten und Fettsäuren, die wohl weniger Einfluß auf die biologische Aktivität als auf nachfolgende Standardisierungs-Verfahren haben dürften (Tabellen 4 und 5). Auffällig ist allerdings der große $i\text{-}C_{14} : i\text{-}C_{17}$ Quotient, der in

vorangegangenen Experimenten mit der immunstimulatorischen Potenz der untersuchten Substanzen korrelierte und der für zukünftige Standardisierungen von Bedeutung ist.

Tabelle 4

| Zucker: | Arabinose | Fukose | Mannose | Galaktose | Glukose | GalNAc | GlcNAc |
|---------|-----------|--------|---------|-----------|---------|--------|--------|
| Substrat: | | | | | | | |
| KP-40-Zellen | 0,40 | 1,97 | 1,06 | 0,28 | 2,77 | 3,02 | 1,22 |
| KP-40-lysat | 1,72 | 2,44 | 0,16 | 0,44 | 0,94 | 1,60 | 1,55 |
| Gaschromatographische Analyse der verschiedenen Kohlenhydrate ($\mu$g), die in 1 mg der Proben (KP-40-Zellen bzw.-Lysat) enthalten sind. (GalNAc = N-Acetylgalaktosamin; GlcNAc = A-Acetylglukosamin). Die Bestimmungen wurden nach der Methodik von Chaplin (1982) durchgeführt. | | | | | | | |

| Fettsäuren / Substrat | $i\text{-}C_{14}$ | $n\text{-}C_{14}$ | $i\text{-}C_{15}$ | $a\text{-}C_{15}$ | $n\text{-}C_{15}$ | $i\text{-}C_{16}$ | $n\text{-}C_{16}$ | $i\text{-}C_{17}$ | $a\text{-}C_{17}$ | $n\text{-}C_{17}$ | $i\text{-}C_{18}$ | $\dfrac{i\text{-}C_{14}}{i\text{-}C_{17}}$ |
|---|---|---|---|---|---|---|---|---|---|---|---|---|
| KP-40-Zellen | 40.0 | 12.2 | 178.4 | 66.7 | 22.3 | 8.1 | 10.0 | 21.3 | 8.2 | 4.3 | – | 1.87 |
| KP-40-Lysat | 43.7 | 12.9 | 201.4 | 71.7 | 24.6 | 9.4 | 12.5 | 21.5 | 21.3 | 7.5 | 4.3 | 2.05 |

Tabelle 5:

Gaschromatographische Analyse der Fettsäuren von Propionibakterien KP-40-Zellen und -Lysat (nach Minnikin et al., 1980; Bestimmung der "peak area" durch Multiplikation von Peakhöhe und Retentionszeit). Der Quotient $i\text{-}C_{14}$ : $i\text{-}C_{17}$ korrelierte in vorangegangenen Experimenten mit der immunstimulatorischen Potenz von Substraten. Werte > 1 waren charakteristisch für gute Aktivatoren.

EP 0 343 544 A2

Das Propionibacterium KP-40-Lysat übt in vitro und in vivo eine ausgezeichnete immunstimulierende Wirkung aus. Es ist als Alternative zur bereits bekannten Immuntherapie mit ganzen, hitzeinaktivierten Propionibacterium avidum KP-40-Zellen bzw. Propionibacterium granulosum KP-45-Zellen zu empfehlen. Die Möglichkeit der subkutanen und eventuellen lokalen Applikation über die Schleimhäute eröffnet ferner ein breites Anwendungsspektrum.

Die Erfindung wird anhand der folgenden Beispiele näher erläutert.

**Beispiel 1**

Für die Massenanzüchtung der Propionibakterien wurden A-Agarplatten mit einer dreitägigen A-Agarvorkultur beimpft und im anaeroben Brutschrank (in einer 5% $CO_2$, 10% $H_2$ 85% $N_2$-Atmosphäre) bei 37°C inkubiert. Zur Kultur im anaeroben Milieu wurde sowohl das Gas-Pack-Topf-Verfahren (BBL) als auch der Anaerobenbrutschrank (Heraeus) angewandt. Eine optimale Zellausbeute konnte aus den Massenkulturen nach 72 Stunden Bebrütungsdauer erhalten werden. Danach wurden die Kulturplatten mit 2 bis 3 ml physiologischer Kochsalzlösung überflutet und nach etwa 10 Minuten mit der physiologischen Lösung von den A-Agarkultur-Oberflächen abgespatelt. Die Zellen wurden 20 Minuten durch Zentrifugieren bei 9000 U/min in einer Sorval R 2B-Kühlzentrifuge geerntet und zweimal mit physiologischer Kochsalzlösung gewaschen.

Zwecks Herstellung des immunmodulatorischen Lysats wurden die KP-40-Zellen bzw. die KP-45-Zellen mit dem doppelten Volumen Glasperlen (Durchmesser 0,17 bis 18 mm) vermischt und in einer Zellmühle (Vibrogen-Zellmühle Vi3, E. Bühler, Tübingen) 1 bis 1,5 Stunden zerschlagen.

Nachdem durch eine mikroskopische Kontrolle (Gram-Präparat) geprüft worden war, daß keine ganzen Zellen mehr vorhanden waren, wurden die Glasperlen mit einer G-1-Fritte unter Verwendung von destilliertem Wasser vom Homogenisat abgetrennt. Die milchige Suspension wurde 30 Minuten bei 15000 U/min zentrifugiert, der Überstand dekantiert, 30 Minuten im Dampftopf erhitzt, abermals 30 Minuten zentrifugiert, durch Millipore (0,45 μm) filtriert und gefriergetrocknet.

**Beispiel 2**

Herstellen des immunstimulierenden Mittels aus den Propionibakterienstämmen Propionibacterium granulosum KP-45 und Propionibacterium avidum KP-40

Massenkulturen der Propionibakterienstämme (etwa 30 g Naßgewicht der Zellen) wurden auf 100 ml Agua dest. aufgefüllt und mit der gleichen Menge 95%-iger Phenollösung gemischt. Diese Mischung wurde bei Raumtemperatur über 2 Stunden gerührt. Danach wurde diese Mischung für 40 Minuten bei 5000 U/min zentrifugiert, die wäßrige Phase abpipettiert und gesammelt. Das Sediment und die Phenolphase wurden nochmals in 100 ml Agua dest. gelöst, eine weitere Stunde bei Raumtemperatur gerührt, abzentrifugiert und die wäßrige Phase abpipettiert und gesammelt. Die wäßrige Phase wurde anschließend 3 Tage bei 4°C gegen Agua dest., das mehrmals gewechselt wurde, dialysiert und anschließend gefriergetrocknet.

**Beispiel 3**

Wirksamkeitsuntersuchungen

Für die Tierexperimente wurden ausschließlich männliche, 8 bis 12 Wochen alte Balb/c-Inzuchtmäuse verwendet (Herkunft: Zentralinstitut für Versuchstierzucht, Hannover). Sie wurden in Plastikkäfigen gehalten und hatten freien Zugang zum Futter und Wasser.

Die für die in vitro-Untersuchungen benutzten menschlichen Blutzellen stammten von gesunden Freiwilligen beiderlei Geschlechts. Die Leukozyten wurden wie folgt gewonnen: 30 bis 40 ml Blut, welches 10 I.U./ml zusatzfreies Heparin (Sigma Chemicals Co., St. Louis) enthielt, wurden mit 6% Dextranlösung in 0,9%-iger Kochsalzlösung (ein Viertel des obigen Volumens) vermischt, die roten Blutkörperchen konnten anschließend 30 bis 45 Minuten bei Zimmertemperatur sedimentieren. Durch Zentrifugieren des Überstandes (10 Minuten bei 350 g) wurde ein leukozytenreiches Sediment hergestellt, welches dann weiterverwendet wurde. Die eventuell noch verbliebenen Erythrozyten wurden durch kurze Zugabe (45 Sekunden) von 0,2%-iger Kochsalzlösung zum Sediment lysiert, dann sofort gefolgt von einer gleichvolumigen Zugabe von 1,6%-iger Kochsalzlösung. Die Leukozyten wurden zweimal in Hanks Balanced Salt Solution (HBSS, Gibco Lab., Karlsruhe) gewaschen und anschließend in der gewünschten Konzentration in Minimal Essential Medium (MEM, Gibco Lab.) resuspendiert, welches Hepes Puffer und 1% hitzeinaktiviertes Kälberserum enthielt.

Zur Gewinnung von Monozyten wurde das Blut mit 0,9%-iger Kochsalzlösung im Verhältnis 1:1 gemischt, über einen Ficoll-Hypaque Density Gradienten geschickt und bei Zimmertemperatur zentrifugiert (40 Minuten bei 1400 g). Die Monozytenschicht wurde gesammelt, zweimal in HBSS gewaschen und in einer Konzentration von $5 \times 10^6$ lebenden Zellen/ml in Zellkulturmedium RPMI 1640 (Gibco Lab.) resuspendiert, welches mit 10% autologem Serum angereichert war. Diese Suspension wurde für eine Stunde bei $37^\circ$ C in mit autologem Serum beschichteten Platten bebrütet. Nach Entfernen der nichtadhärenten Zellen wurden die anhaftenden Zellen losgelöst. Die so gewonnene Zellpräparation enthielt 90% Monozyten (mit Neutralrotfärbung bestimmt).

Zur Gewinnung von Peritonealmakrophagen der Maus wurden Balb/c-Mäuse getötet, laparatomiert und die Bauchhöhle vorsichtig mit HBSS (Gibco Lab.), die 10 I.U. Heparin (Sigma Chemicals Co.) enthielt, gespült. Die Spülflüssigkeit wurde zentrifugiert (10 Minuten, 400 x g) und die Zellen resuspendiert in HBSS, versetzt mit 10% hitzeinaktiviertem fetalen Kälberserum. Es wurde eine Zellsuspension hergestellt ($5 \times 10^6$/ml), und 2 ml dieser Suspension wurden in sterile Petri-Schalen (Falcon, Becton-Dickinson, Heidelberg) überführt und für 2 Stunden inkubiert ($37^\circ$ C, 5% $CO_2$). Nach Dekantierung des Überstandes wurden die adhärierenden Makrophagen zweimal mit HBSS (Gibco Lab.) gewaschen, losgelöst und in MEM (Gibco Lab.), versetzt mit 1% fetalem Kälberserum, für Chemilumineszenz-Messungen suspendiert. Nach Prüfung der Vitalität 95%) mit Trypan Blau erfolgte die Auszählung (Hämozytometer) und Verdünnung der vitalen Zellen auf die gewünschte Konzentration von $5 \times 10^6$/ml.

## Beispiel 4

Luminol oder Lucigenin (Sigma Chemicals Co.) wurden in Dimethylsulfoxid (DM50, Sigma Chemicals Co.) in einer Konzentration von $2 \times 10^{-3}$ M aufgelöst. Diese Ausgangslösung wurde in Gewebskulturmedium vor Anwendung weiter verdünnt. Die Reaktionsmischung bestand aus 0,5 ml Zellsuspension ($1 \times 10^6$ Leukozyten/ml, $2,5 \times 10^5$ Monozyten/ml oder $5 \times 10^6$/ml Peritonealmakrophagen) 0, 9 ml Medium mit $2 \times 10^{-6}$ M Luminol oder Lucigenin und 0,1 ml Aktivatorsuspension bzw. -lösung, wie unter Versuchsschema erläutert. Alle Bestandteile dieser Reaktionsmischung wurden bei $37^\circ$ C in einem Wasserbad vorgewärmt und unmittelbar vor Testbeginn zusammengegeben. Die Messung der Chemilumineszenz wurde bei $37^\circ$ C mit dem LKB Wallac-Luminometer 1250 durchgeführt (Angabe in Millivolt, mV).

Gaschromatographie: Die gaschromatographischen Analysen wurden mit dem Gerät Nr. 7400 der Firma Hewlett-Packard durchgeführt. Dieses ist ausgestattet mit zwei Flammenionisationsdetektoren und Glassäulen von 3 m Länge und einem Innendurchmesser von 0,2 mm. Die Bedingungen der gaschromatographischen Kohlenhydratanalyse nach Chaplin, M.F., Anal. Biochem. 123, 336-341 (1982) waren:

1. Trägergas: Stickstoff; der Durchfluß betrug 50 ml/min.
2. Temperaturprogramm: $4^\circ$ C/min von 160 bis $260^\circ$ C
3. Detektortemperatur: $260^\circ$ C
4. Injektionstemperatur: $250^\circ$ C
5. Proben: 3 $\mu$l mit einer Thermomikrospitze (Volumen 10 $\mu$l).

Die Proben wurden 3 Minuten bei der Anfangstemperatur gehalten, bevor das Temperaturprogramm begann. Die gaschromatographische Fettsäurebestimmung erfolgte nach der Methodik von Minnikin, D.E. et al., J. Chrom. 188, 221-233 (1980).

Chemotaxis: Die Chemotaxis der menschlichen Granulozyten wurde nach der Methode von Chenoweth D. E. et al., J. Immunol. Methods 25, 337-352 (1979) untersucht. Eine spontane wie auch chemotaktische Wanderung der Zellen unter Agarose wurde mit einem Mikroskalenokular gemessen und der Koeffizient der Migration (MC) bestimmt, und zwar mit folgender Formel:

$$MC= \frac{CD \times MD}{\sqrt{Granulozytenkonzentration}}$$

CD = Chemotaxis-Differenz (chemotaktische minus spontane Migration)
MD = Migrationsdichte, ausgedrückt in einer Skala von 1 bis 4.

## Beispiel 5

Tumor: Für die in vivo-Experimente wurde das L-1 Sarkom verwendet, ein in der Lunge einer Balb/c-Maus spontan entstandener und in dieser Tierart über 120 bis 130 Passagen gehaltener solider Tumor. Ein 14 Tage nach subkutaner Applikation von 1 x $10^5$ vitalen Tumorzellen, suspendiert in 0,1 ml PBS (phosphate buffered saline, Flow Laboratories, Meckenheim), am Inokulationsort entstandener Tumor wurde enukleiert, in kleine Stücke zerlegt und vorsichtig durch ein Stahlsieb gerieben. Die separierten L-1 Zellen wurden dreimal in PBS gewaschen, in Nährmedium RPMI 1640 (Gibco Lab.) aufgenommen, gezählt und mit Trypan-Blau auf ihre Vitalität geprüft. Alle Experimente wurden mit vitalen, vollständig disaggregierten L-1 Zellen ausgeführt, die in 0,1 ml PBS suspendiert und intravenös (in die Schwanzvenen der Balb/c-Mäuse) appliziert wurden. Die intravenöse Gabe der L-1 Tumorzellen führte zu einer ausschließlichen Manifestation des Tumors in den Lungen der Versuchstiere, die entsprechend der Methode von Hill und Bush gezählt wurden (Int. J. Radiat. Biol. 15, 435-444 (1969)).

## Beispiel 6

Das immunmodulatorische Propionibacterium KP-40-Lysat bzw. Propionibacterium KP-45-Lysat wurde bei allen in vivo-Experimenten den Balb/c-Mäusen 7, 4 und 2 Tage vor Testbeginn in Dosierungen von 1, 2,5 und 5 mg (gelöst in 0,1 oder 0,2 ml PBS) intraperitoneal, subkutan oder nasal verabreicht. Als Kontrolle fungierende Mäusegruppen erhielten anstelle des Lysats gleiche Volumina von physiologischer Kochsalzlösung. Getestet wurde die Auswirkung des Lysats auf die Aktivierbarkeit von Maus-Peritonealmakrophagen durch serum-aktiviertes Zymosan (Chemilumineszenz-Test) auf das Milzgewicht (als Parameter für die immunstimulatorische Wirksamkeit; bezogen auf 20 g Körpergewicht) und auf die Tumor-protektive Wirkung (Anzahl der Tumorkolonien in den Versuchstierlungen) Für die in vitro-Untersuchungen (Chemilumineszenz-Test menschlicher Monozyten und Granulozyten, Chemotaxis) wurden Lysat-Konzentrationen von 2,5 und 5 mg lyophilisiert/ml PBS verwendet.

## Ansprüche

1. Immunstimulierendes Mittel aus Propionibakterien, insbesondere aus den Stämmen Propionibacterium granulosum DSM 1773, Propionibacterium avidum DSM 1772 und/oder Propionibacterium acnes ATCC 6919, dadurch gekennzeichnet, daß es aus dem wäßrigen Zell-Lysat, vorzugsweise aus dem mit organischen Lösungsmitteln extrahierten Zell-Lysat besteht und gewünschtenfalls dialysiert und gefriergetrocknet ist.

2. Mittel gemäß Anspruch 1, dadurch gekennzeichnet, daß es aus Lipoteichonsäuren (LTA) besteht, die aus dem wäßrigen Lysat isoliert und gereinigt wurden.

3. Verfahren zur Herstellung eines Mittel nach Anspruch 1 oder 2, dadurch gekennzeichnet, daß die Kulturen der Propionibakterienstämme lysiert und die Zelltrümmer abgetrennt werden.

4. Verfahren nach Anspruch 3, dadurch gekennzeichnet, daß die Abtrennung der Zelltrümmer durch Zentrifugation oder Filtration geschieht.

5. Verfahren nach Anspruch 3 oder 4, dadurch gekennzeichnet, daß das wäßrige Lysat mit organischen Extraktionsmitteln behandelt und die wäßrige Phase isoliert wird.

6. Verfahren nach Anspruch 5, dadurch gekennzeichnet, daß das organische Extraktionsmittel eine wäßrige Phenollösung ist.

7. Verfahren nach einem der Ansprüche 3 bis 5, dadurch gekennzeichnet, daß die wäßrige Phase gegen Wasser dialysiert und gewünschtenfalls gefriergetrocknet wird.

8. Verfahren nach einem der Ansprüche 3 bis 7, dadurch gekennzeichnet, daß die wäßrige Phase in Fraktionen aufgetrennt wird und die die Lipoteichonsäuren (LTA) enthaltenden Fraktionen gesammelt und gegebenenfalls dialysiert und gefriergetrocknet werden.

9. Verwendung des Mittels nach Anspruch 1 oder 2 als Immunstimulans.

10. Verwendung des Mittels nach Anspruch 1 oder 2 zwecks unterstützender Maßnahmen in der Krebstherapie und -prophylaxe sowie der Rezidiv- und Metastasenvorbeugung.

11. Verwendung des Mittels nach Anspruch 1 oder 2 als Unterstützung bei chirurgischen Eingriffen.

12. Verwendung des Mittels nach Anspruch 1 oder 2 zur Bekämpfung und Prophylaxe viraler, mykologischer und bakterieller Infektionen sowie parasitologischer Erkrankungen.

13. Verwendung des Mittels nach Anspruch 1 oder 2 zur Herstellung eines Nasensprays.

14. Verwendung des Mittels nach Anspruch 1 oder 2 zur Herstellung einer Infusions- oder Injektionslösung.

15. Verwendung des Mittels nach Anspruch 1 oder 2 zur Herstellung eines oral zu verabreichenden Mittels in Form von Kapseln oder Lösung.

16. Verwendung des Mittels nach Anspruch 1 oder 2 zur Herstellung von Suppositorien.